(19) Europäisches Patentamt
European Patent Office
Office européen des brevets

(11) Numéro de publication: **0 497 697 B1**

# (12) FASCICULE DE BREVET EUROPEEN

(45) Date de publication du fascicule du brevet: **06.12.95**

(51) Int. Cl.$^6$: **A61K 7/13**

(21) Numéro de dépôt: **92400237.1**

(22) Date de dépôt: **30.01.92**

**(54) Procédé de teinture des fibres kératiniques, associant l'isatine ou ses dérivés à un amino indole ou une amino indoline, compositions mises en oeuvre.**

(30) Priorité: **01.02.91 FR 9101186**

(43) Date de publication de la demande:
**05.08.92 Bulletin 92/32**

(45) Mention de la délivrance du brevet:
**06.12.95 Bulletin 95/49**

(84) Etats contractants désignés:
**AT BE CH DE DK ES FR GB GR IT LI NL PT SE**

(56) Documents cités:
**EP-A- 0 359 465**
**US-A- 4 013 404**

(73) Titulaire: **L'OREAL**
**14, rue Royale**
**F-75008 Paris (FR)**

(72) Inventeur: **Lang, Gérard**
**44, avenue Lacour**
**F-95210 Saint-Gratien (FR)**
Inventeur: **Cotteret, Jean**
**15, Allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne un procédé de teinture des fibres kératiniques, en particulier des cheveux humains, associant l'isatine ou l'un de ses dérivés à un amino indole ou une amino indoline, ainsi que les compositions mises en oeuvre.

En coloration directe des cheveux, c'est-à-dire dans un procédé de teinture ne mettant pas en oeuvre un processus de développement des colorants par voie oxydative, on a déjà proposé d'utiliser l'isatine comme colorant jaune de base dans le brevet français n° 2.588.473.

La demande européenne n° 0359.465 a proposé ensuite un procédé de teinture directe utilisant l'isatine ou l'un de ses dérivés en association avec des dérivés d'aminobenzène disubstitués.

La demanderesse vient de découvrir, d'une manière surprenante, un nouveau procédé de teinture associant l'isatine ou ses dérivés à des colorants du type amino indole ou amino indoline, permettant d'obtenir une large gamme de nuances plus résistantes aux shampooings et à la transpiration que celles obtenues avec les procédés de teinture directe utilisant les dérivés aminés connus de l'art antérieur. Les colorations obtenues sont de plus stables à la lumière, aux intempéries et aux agents chimiques.

La présente invention a donc pour objet un procédé de teinture des fibres kératiniques, consistant à appliquer sur les fibres, l'isatine ou l'un de ses dérivés, et un amino indole ou une amino indoline, soit simultanément sous forme d'un mélange extemporané, soit de façon successive.

Un objet de l'invention consiste également en un agent de teinture à deux composants.

D'autres objets apparaîtront à la lumière de la description.

Le procédé de teinture des fibres kératiniques, en particulier des cheveux humains, conforme à la présente invention, est caractérisé essentiellement par le fait qu'il comporte l'application sur lesdites fibres d'un composant (A) constitué d'une composition contenant dans un milieu approprié pour la teinture, au moins un composé de formule (I) suivante :

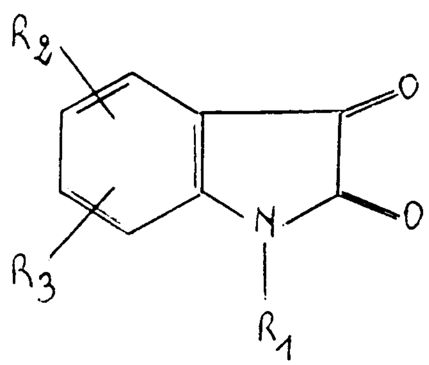

(I)

dans laquelle :

$R_1$ désigne un atome d'hydrogène, un radical alkyle en $C_1$-$C_6$, acétyle, benzoyle, phényle ou carboxyalkyle en $C_1$-$C_4$;

$R_2$ et $R_3$, indépendamment l'un de l'autre, désignent un atome d'hydrogène, un alkyle en $C_1$-$C_6$, un alcoxy en $C_1$-$C_4$, un hydroxyle, un amino, un atome d'halogène, un groupement nitro, un alkyl($C_1$-$C_6$)-phényle, phényle, alkyl($C_1$-$C_6$)amino, hydroxyalkyl($C_1$-$C_6$)amino, polyhydroxyalkyl($C_2$-$C_6$)amino;

et un composant (B) constitué d'une composition contenant dans un milieu approprié pour la teinture, au moins un amino indole de formule (II) suivante :

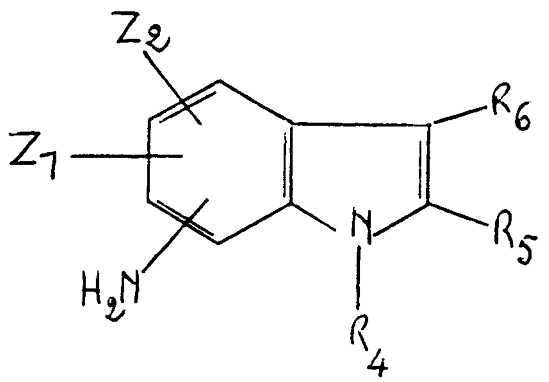

(II)

dans laquelle :

$R_4$ et $R_6$, indépendamment l'un de l'autre, représentent un atome d'hydrogène ou un groupement alkyle en $C_1$-$C_4$;

$R_5$ désigne un atome d'hydrogène ou un groupement alkyle en $C_1$-$C_4$, COOR', R' étant un atome d'hydrogène ou un groupement alkyle en $C_1$-$C_4$;

$Z_1$ représente un atome d'hydrogène, d'halogène, un radical alkyle en $C_1$-$C_4$, alcoxy en $C_1$-$C_4$ ou hydroxyle;

$Z_2$ représente un atome d'hydrogène ou un radical alkyle en $C_1$-$C_4$;

ainsi que ses sels cosmétiquement acceptables, ou bien une amino indoline de formule (III) :

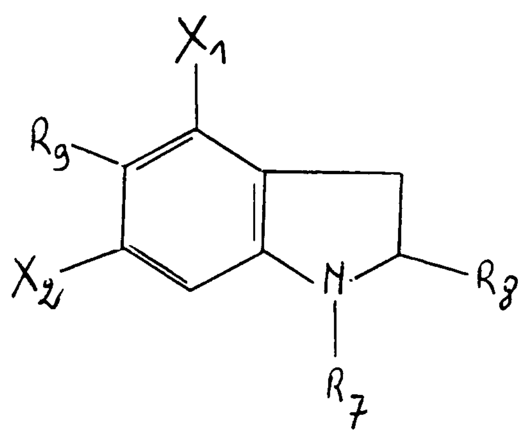

(III)

dans laquelle :

$R_7$ et $R_8$, indépendamment l'un de l'autre, représentent un atome d'hydrogène ou un radical alkyle en $C_1$-$C_4$;

$X_1$ et $X_2$ représentent un atome d'hydrogène ou un radical $NH_2$, au moins un et un seul représentant $NH_2$;

$R_9$ désigne un atome d'hydrogène, un radical alkyle en $C_1$-$C_4$, alcoxy en $C_1$-$C_4$; $R_9$ désignant hydrogène lorsque $X_2$ désigne $NH_2$;

ainsi que ses sels cosmétiquement acceptables.

Le procédé selon l'invention peut être mis en oeuvre sans l'intervention d'un agent oxydant autre que l'air.

Le procédé de teinture décrit ci-dessus conduit à la formation d'une base de Schiff, soit lors du mélange de la composition (A) avec la composition (B), soit in situ dans la fibre kératinique lors d'une application séquentielle des compositions (A) et (B). Cette base de Schiff a pour formule :

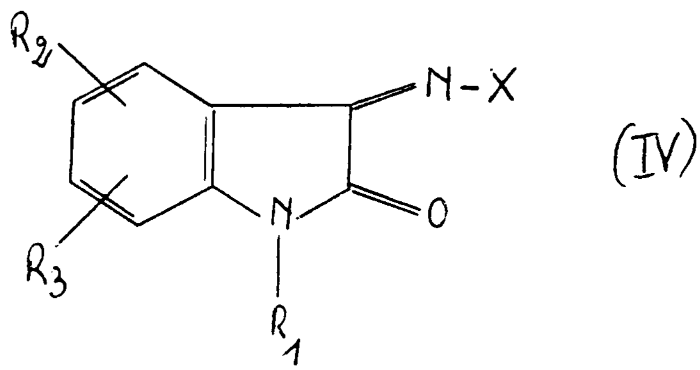

dans laquelle X désigne :

a)

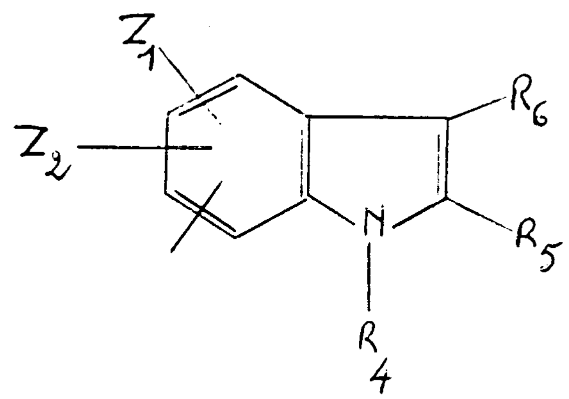

ou b)

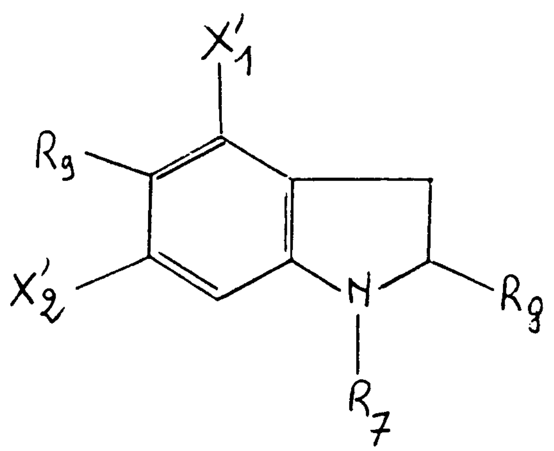

$R_1$ à $R_9$, $Z_1$ et $Z_2$ ont les significations indiquées ci-dessus pour les formules (I), (II) et (III);

$X'_1$ et $X'_2$ désignent un atome d'hydrogène ou une liaison covalente, au moins un et un seul désignant un atome d'hydrogène;

$R_9$ désignant hydrogène lorsque $X'_2$ représente une liaison covalente.

Parmi les composés de formule (I), on peut citer plus particulièrement l'isatine.

Les composés de formule (II) préférentiels sont choisis parmi :

le 4-aminoindole

le 5-aminoindole

le 7-aminoindole

le 2,3-diméthyl 5-amino 6-hydroxyindole

le 2,3-diméthyl 5-amino 6-méthoxyindole

le 2,3-diméthyl 5-chloro 6-aminoindole

le 2,3,4,5-tétraméthyl 6-aminoindole
le 2,3-diméthyl 5-méthoxy 6-aminoindole
le 2,3-diméthyl 5-éthyl 6-aminoindole
le 2-méthyl 6-aminoindole

Les composés de formule (II) plus particulièrement préférés sont choisis parmi :
le 6-aminoindole
le 2,3-diméthyl 5-hydroxy 6-aminoindole
le 2,3,5-triméthyl 6-aminoindole
le 2-méthyl 5-hydroxy 6-aminoindole
le 2,3-diméthyl 6-aminoindole
le 2,3,7-triméthyl 6-aminoindole
le 2,3,4-triméthyl 6-aminoindole

Les composés de formule (III) préférentiels sont choisis parmi la 6-amino indoline et la N-éthyl 6-amino indoline.

Selon le procédé de la présente invention, le composé de formule (I) est de préférence présent dans le composant (A) dans des proportions comprises entre 0,01 et 5% en poids et plus particulièrement entre 0,25 et 2% en poids par rapport au poids total du composant (A) ou des composants (A) + (B) et le composé de formule (II) ou (III) est présent dans le composant (B) dans des proportions comprises de préférence entre 0,01 et 5% en poids et en particulier entre 0,25 et 2% en poids par rapport au poids total du composant (B) ou des composants (A) + (B).

Les compositions (A) et (B) utilisables conformément à l'invention, sont des compositions liquides plus ou moins épaissies, aqueuses ou anhydres, des crèmes, des gels aqueux ou anhydres, des huiles ou des poudres à diluer avec un liquide au moment de l'emploi, encore appelées "cataplasmes".

Dans une première forme de réalisation de l'invention, le milieu cosmétique approprié pour la teinture est aqueux et a un pH pouvant varier entre 2 et 10, et de préférence entre 3 et 9,5, il est ajusté à la valeur désirée à l'aide d'agents alcalinisants ou d'agents acidifiants connus en eux-mêmes.

Ces compositions peuvent contenir des agents tensio-actifs anioniques, cationiques, non-ioniques, amphotères ou leurs mélanges. Ces tensio-actifs sont présents dans les compositions conformes à l'invention dans des proportions comprises entre 0,1 et 55% en poids, et de préférence entre 1 et 40% en poids par rapport au poids total de chaque composition.

Ces compositions aqueuses peuvent renfermer des solvants organiques parmi lesquels on peut mentionner à titre d'exemple, les alcanols inférieurs tels que l'éthanol ou l'isopropanol, les polyols tels que le glycérol, les glycols ou éthers de glycols comme l'éthylène glycol, le propylèneglycol, l'éther monobutylique de l'éthylène glycol, le monoéthyléther et le mononéthyléther de diéthylène glycol ainsi que des produits analogues ou leurs mélanges.

Ces solvants sont de préférence utilisés dans des proportions allant de 1 à 60% en poids, et plus particulièrement de 3 à 30% en poids par rapport au poids total de la composition.

Ces compositions peuvent être épaissies avec des agents choisis parmi l'alginate de sodium, la gomme arabique, la gomme de guar ou de caroube, la gomme de xanthane, les pectines, les dérivés de la cellulose et les polymères divers ayant une fonction épaississante tels que les dérivés d'acide acrylique. On peut également utiliser des agents épaississants minéraux tels que la bentone.

Ces agents épaississants sont présents de préférence dans des proportions comprises entre 0,1 et 5% en poids et en particulier entre 0,5 et 3% en poids par rapport au poids total de la composition.

Ces compositions peuvent également contenir des polymères anioniques, non-ioniques, cationiques, amphotères ou leurs mélanges, en des proportions de 0,1 à 5% en poids par rapport au poids total de la composition.

Ces compositions peuvent bien entendu contenir tous autres adjuvants habituellement utilisés dans les compositions pour la teinture des cheveux, tels que les agents de pénétration, les agents séquestrants, les agents antioxydants, des tampons, des parfums, des colorants, etc...

Une forme préférée de l'invention consiste à utiliser un milieu anhydre tel que décrit dans le brevet français n° 2.526.031.

On entend par milieu anhydre un milieu ne contenant pas plus de 1% d'eau.

Le milieu anhydre est constitué conformément à cette variante de l'invention, par un mélange d'au moins un solvant anhydre et d'un ou plusieurs agents tensio-actifs anhydres, de telle sorte que ces compositions contiennent au moins 15% de solvant et au moins 20% d'agent tensio-actif.

Les solvants utilisés sont des solvants cosmétiquement acceptables choisis parmi les monoalcools saturés en $C_2$-$C_{20}$ tels que l'éthanol, l'isopropanol, l'alcool cétylique ou l'octyldodécanol; les polyols tels que les alcoylèneglycols comme l'éthylèneglycol, le propylèneglycol, le glycérol, le diéthylèneglycol; les

éthers de glycols tels que les mono-, di- et triéthylèneglycolmonoalcoyléthers comme par exemple l'éthylèneglycolmonoéthyléther, l'éthylèneglycolmonobutyléther, le diéthylèneglycolmonoéthyléther; des esters comme par exemple l'acétate de monométhyléther de l'éthylèneglycol, l'acétate de monoéthyléther de l'éthylèneglycol; les esters d'acides gras et d'alcools inférieurs saturés comme le myristate ou le palmitate d'isopropyle.

Les compositions particulièrement préférées contiennent un solvant choisi parmi l'éthanol, l'alcool cétylique, le propylèneglycol, l'éthylèneglycolmonoéthyléther ou l'éthylèneglycolmonobutyléther.

Les agents tensio-actifs utilisés dans cette forme de réalisation sont choisis parmi les agents tensio-actifs anhydres de type anionique, non-ionique, cationique, amphotère ou leurs mélanges. On peut citer plus particulièrement les alcools gras polyoxyéthylénés, les alkylphénols ou naphtols polyoxyéthylénés, les halogénures de monoalkyltriméthylammonium, les halogénures de dialkyldiméthylammonium, les savons, les alcools gras polyglycérolés. Les agents tensio-actifs préférés sont les agents tensio-actifs non-ioniques.

Ces compositions peuvent contenir un agent alcalin ou acidifiant anhydre tel que par exemple l'acide citrique, l'acide ascorbique, l'acide acétique, l'acide lactique et des alcanolamines telles que, de préférence, celles qui sont totalement substituées sur le groupement amine comme le diméthylaminoéthanol.

En-dehors des composés décrits ci-dessus, les compositions anhydres conformes à l'invention peuvent contenir de nombreux additifs utilisables en cosmétique à la seule condition qu'ils contiennent moins de 1% d'eau. Parmi ces additifs, on peut citer les parfums, les agents épaississants, les agents traitants, les agents antioxydants, les huiles végétales ou minérales, les agents conservateurs et les sels organiques.

Ces compositions peuvent être appliquées telles quelles sur les cheveux mouillés ou être diluées tout juste avant l'emploi. Dans ce dernier cas, au moment de la teinture, les compositions selon l'invention sont diluées avec une solution aqueuse, de telle sorte que le rapport entre la composition conforme à l'invention et la solution aqueuse soit compris entre 0,25 et 2. La solution aqueuse peut être constituée par de l'eau pure, mais également par tout autre liquide aqueux complexe plus ou moins épaissi tel que par exemple un support habituellement utilisé dans les compositions tinctoriales pour cheveux.

Dans ce cas, les composants du milieu cosmétique peuvent être tous types d'ingrédients cosmétiquement acceptables, anhydres ou non, habituellement utilisés dans ce type de composition et décrits de façon générale ci-dessus.

Une autre forme d'utilisation des compositions (A) et/ou (B) conformes à l'invention, est constituée par l'utilisation sous forme de cataplasmes, c'est-à-dire sous forme de poudre à diluer avec un liquide au moment de l'emploi.

Dans cette forme de réalisation, les colorants sont préparés sous forme de poudre stable au stockage et introduits dans un milieu solide pouvant être constitué de poudres, de farines, de substances amylacées ou mucilagineuses que l'on dilue au moment de l'emploi avec un liquide adéquat de façon à former un mélange ayant une consistance appropriée pour être appliqué sur tête.

Les poudres ou farines utilisées dans ce type de composition sont constituées généralement par des substances insolubles telles que des silices, des argiles, des végétaux pulvérisés après extraction de leurs principes actifs par solvant.

Le liquide peut être constitué par de l'eau ou des mélanges d'eau et de solvants cosmétiquement acceptables tels que des alcools ou des glycols ou encore par des huiles.

Le milieu liquide est additionné à la poudre dans des proportions telles qu'après mélange, on obtienne une pâte ayant une viscosité comprise entre 0,3 et 5 Pa.s.

Un objet de l'invention est constitué par un agent de teinture pour les fibres kératiniques, en particulier des cheveux humains, caractérisé par le fait qu'il est constitué par les composants (A) et (B) stockés sous forme séparée, tels que définis ci-dessus

Les composants (A) et (B) sont destinés, soit à être mélangés tout juste avant emploi, soit à être appliqués de façon successive sur les fibres à traiter.

Selon une forme de réalisation, on peut conditionner les différents composants (A) et (B) dans un dispositif à plusieurs compartiments encore appelé "kit de teinture" comportant tous les composants destinés à être appliqués pour une même teinture sur les fibres kératiniques, en particulier les cheveux, en applications successives avec ou sans prémélange.

De tels dispositifs peuvent comporter un premier compartiment contenant la composition (A) renfermant l'isatine ou ses dérivés de formule (I) et un second compartiment comportant la composition (B) renfermant l'amino indole de formule (II) ou l'amino indoline de formule (III).

Une autre variante peut également consister à stocker la composition (A) ou la composition (B) dans un milieu solvant anhydre et à prévoir un troisième compartiment contenant un milieu aqueux approprié pour la teinture et cosmétiquement acceptable. Dans ce cas, on mélange tout juste avant l'emploi le contenu du troisième compartiment dans l'un ou l'autre ou les deux compartiments contenant les compositions

anhydres (A) et (B) ou alors on mélange avant emploi les trois compartiments.

Selon une variante, le procédé de l'invention consiste à mélanger juste avant l'emploi la composition (A) à la composition (B), la composition résultante étant appliquée sur les cheveux pendant 5 à 40 minutes et de préférence 20 à 30 minutes. Les cheveux sont ensuite rincés, lavés au shampooing, rincés à nouveau puis séchés.

Selon une autre variante, le procédé de l'invention consiste à appliquer sur les cheveux au moins une composition (A) et une composition (B) telles que définies ci-dessus; à laisser poser chacune d'entre elles pendant 5 à 40 minutes, de préférence 20 à 30 minutes, à éventuellement rincer à l'eau entre les deux étapes. Les cheveux sont ensuite rincés, lavés au shampooing, rincés à nouveau puis séchés.

Les exemples suivants sont destinés à illustrer l'invention sans pour autant présenter un caractère limitatif.

EXEMPLES 1 à 9

On procède à la teinture des cheveux en appliquant sur des cheveux naturels gris à 90% de blancs, 20 g des compositions.

Les compositions sont préparées juste avant l'emploi. La composition de l'exemple 9 est mélangée avec 1,5 fois son poids en eau au moment de l'emploi (pH9).

On laisse agir la composition pendant 20 minutes, puis on rince les cheveux, on effectue un shampooing puis on rince à nouveau. Après séchage, les cheveux sont teints dans la nuance précisée au bas du tableau ci-après.

TABLEAU I

| en g MA | 1 | 2 | 3 | 4 | 5 |
|---|---|---|---|---|---|
| Isatine | 1 | 1 | 1 | 1 | 1 |
| 6-aminoindole | 1 | | | | |
| 2,3-diméthyl 5-hydroxy 6-aminoindole, 2 HBr | | 1 | | | |
| 2,3,5-triméthyl 6-aminoindole | | | 1 | | |
| 2,3,7-triméthyl 6-aminoindole | | | | 1 | |
| 2,3,4-triméthyl 6-aminoindole | | | | | 1 |
| Alcool éthylique | 30 | 30 | 30 | 30 | 30 |
| Triéthanolamine qs pH | | 7,5 | 7,5 | | 7,3 |
| pH spontané | 8,1 | | | 7,5 | |
| Eau qsp | 100 | 100 | 100 | 100 | 100 |
| **Nuances obtenues** | cuivré puissant | violine | cuivré rouge | blond irisé beige | irisé rouge |

TABLEAU I (suite)

| en g MA | 6 | 7 | 8 | 9 |
|---|---|---|---|---|
| Isatine | 1 | 1 | | 1 |
| 6-bromoisatine | | | 1 | |
| 2-méthyl 5-hydroxy 6-aminoindole | | 1 | | |
| 2,3-diméthyl 6-aminoindole, HCl | 1 | | 1 | 1 |
| Alcool éthylique | 30 | 30 | 30 | 28,5 |
| Nonylphénol oxyéthyléné à 9 moles d'oxyde d'éthylène qsp | | | | 100 |
| N,N-diméthylamino 2-éthanol | | | | 1 |
| Triéthanolamine qs pH | 7,5 | 7,5 | 8 | |
| Eau qsp | 100 | 100 | 100 | |
| **Nuances obtenues** | acajou rouge | acajou | irisé cendré | blond clair doré |

EXEMPLE 10

Au moment de l'emploi, on mélange la composition avec deux fois son poids en eau. On applique 20 g de ce mélange (pH9,3) sur des cheveux naturels gris à 90% de blancs.

On laisse agir la composition pendant 20 minutes, puis on rince les cheveux, on effectue un shampooing puis on rince à nouveau. Après séchage, les cheveux sont teints dans la nuance précisée au bas du tableau II ci-après.

EXEMPLE 11

Au moment de l'emploi, on mélange les compositions (A) et (B) avec deux fois leur poids en eau. On applique 20 g de la composition (A) (pH8,6) sur 3 g de cheveux naturels gris à 90% de blancs.

On laisse agir la composition pendant 15 minutes, on rince puis on applique 20 g de la composition (B) (pH9,2) pendant 15 minutes, on rince les cheveux, on effectue un shampooing puis on rince à nouveau. Après séchage, les cheveux sont teints dans la nuance précisée au bas du tableau II ci-après.

## TABLEAU II

| en g MA | 10 | 11 | |
|---|---|---|---|
| | | Comp. (A) | Comp. (B) |
| Isatine | 4 | 4 | |
| 2,3-diméthyl 5-hydroxy 6-aminoindole | | | 4 |
| 2,3-diméthyl 6-aminoindole, HCl | 4 | | |
| Gomme de caroube vendue sous la dénomination VIDOGUM L 175 par la Société SANOFI BIO INDUSTRIE | 3 | 3 | 3 |
| Carbonate de calcium | 8 | 8 | 8 |
| Poudre de résidus d'épuisement de saponaire de granulométrie inférieure à 90 microns | 35 | 35 | 35 |
| Lait écrémé en poudre qsp | 100 | 100 | 100 |
| Temps de pose (en mn) | 15 | 15 | 15 |
| **Nuances obtenues** sur cheveux gris à 90% blanc | Rose | blond irisé | |

**TABLEAU II (suite)**

| en g MA | 12 | 13 | 14 | 15 | 16 | 17 | 18 |
|---|---|---|---|---|---|---|---|
| Isatine | 1 | 1 | 1 | 1 | 1 | 1 | 1 |
| 2,3-diméthyl 5-éthyl 6-aminoindole, HCl | 1 | | | | | | |
| 2,3,4,5-tétraméthyl 6-aminoindole, HCl | | 1 | | | | | |
| 5-aminoindole | | | 1 | | | | |
| 7-aminoindole | | | | 1 | | | |
| 2,3-diméthyl 6-hydroxy 5-aminoindole | | | | | 1 | | |
| 2,3-diméthyl 6-méthoxy 5-aminoindole, HCl | | | | | | 1 | |
| 6-aminoindole | | | | | | | 1 |
| Alcool éthylique | 30 | 30 | 30 | 30 | 30 | 30 | 10 |
| Triéthanolamine qs pH | 7,5 | 8,4 | | | | 7,5 | 7,5 |
| pH spontané | | | 8,5 | 7 | 9 | | |
| Eau qsp | 100 | 100 | 100 | 100 | 100 | 100 | 100 |
| **Nuances obtenues** | rouge légèrement cuivré | blond doré cendré légèrement irisé | cuivré doré | beige cuivré doré | cuivré irisé | cuivré doré | légèrement doré |

**Revendications**

**1.** Procédé de teinture des fibres kératiniques, en particulier des cheveux humains, caractérisé par le fait que l'on applique sur lesdites fibres un composant (A) constitué d'une composition renfermant dans un milieu approprié pour la teinture, au moins un composé de formule (I) :

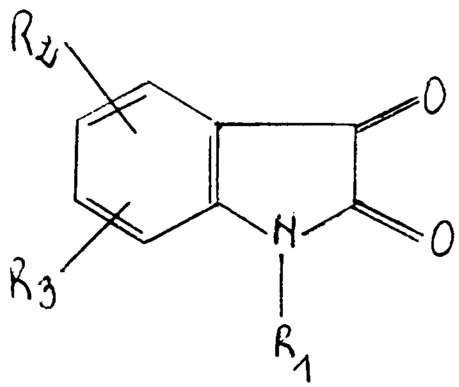

(I)

dans laquelle :

$R_1$ désigne un atome d'hydrogène, un radical alkyle en $C_1$-$C_6$, acétyle, benzoyle, phényle ou carboxyalkyle en $C_1$-$C_4$;

$R_2$ et $R_3$, indépendamment l'un de l'autre, désignent un atome d'hydrogène, un alkyle en $C_1$-$C_6$, un alcoxy en $C_1$-$C_4$, un hydroxyle, un amino, un atome d'halogène, un groupement nitro, un alkyl($C_1$-$C_6$)phényle, phényle, alkyl($C_1$-$C_6$)amino, hydroxyalkyl($C_1$-$C_6$)amino, polyhydroxyalkyl($C_2$-$C_6$)amino;

et un composant (B) constitué d'une composition contenant dans un milieu approprié pour la teinture, au moins un composé amino indole de formule (II) suivante :

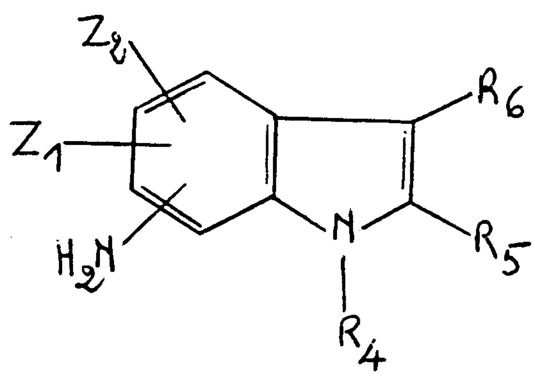

(II)

dans laquelle :

$R_4$ et $R_6$, indépendamment l'un de l'autre, représentent un atome d'hydrogène ou un groupement alkyle en $C_1$-$C_4$;

$R_5$ désigne un atome d'hydrogène ou un groupement alkyle en $C_1$-$C_4$, COOR', R' étant un atome d'hydrogène ou un groupement alkyle en $C_1$-$C_4$;

$Z_1$ représente un atome d'hydrogène, d'halogène, un radical alkyle en $C_1$-$C_4$, alcoxy en $C_1$-$C_4$ ou hydroxyle;

$Z_2$ représente un atome d'hydrogène ou un radical alkyle en $C_1$-$C_4$;

ainsi que ses sels cosmétiquement acceptables, ou bien une amino indoline de formule (III) :

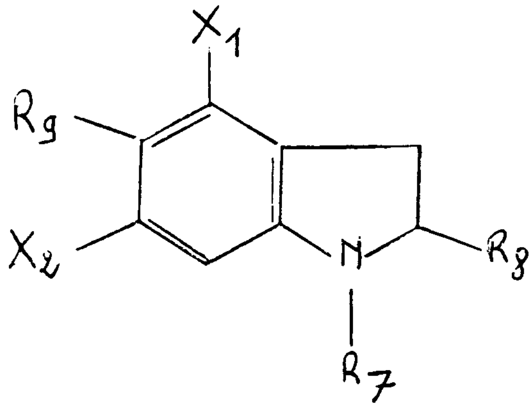

(III)

dans laquelle :

$R_7$ et $R_8$, indépendamment l'un de l'autre, représentent un atome d'hydrogène ou un radical alkyle en $C_1$-$C_4$;

$X_1$ et $X_2$ représentent un atome d'hydrogène ou un radical $NH_2$, au moins un et un seul représentant $NH_2$;

$R_9$ désigne un atome d'hydrogène, un radical alkyle en $C_1$-$C_4$, alcoxy en $C_1$-$C_4$; $R_9$ désignant hydrogène lorsque $X_2$ désigne $NH_2$;

ainsi que ses sels cosmétiquement acceptables.

**2.** Procédé selon la revendication 1, caractérisé par le fait que le composé de formule (I) est l'isatine.

**3.** Procédé selon la revendication 1 ou 2, caractérisé par le fait que le composé de formule (II) est choisi parmi :

le 4-aminoindole
le 5-aminoindole
le 7-aminoindole
le 2,3-diméthyl 5-amino 6-hydroxyindole
le 2,3-diméthyl 5-amino 6-méthoxyindole
le 2,3-diméthyl 5-chloro 6-aminoindole
le 2,3,4,5-tétraméthyl 6-aminoindole
le 2,3-diméthyl 5-méthoxy 6-aminoindole
le 2,3-diméthyl 5-éthyl 6-aminoindole
le 2-méthyl 6-aminoindole.

**4.** Procédé selon la revendication 1 ou 2, caractérisé par le fait que le composé de formule (II) est choisi parmi :

le 6-aminoindole
le 2,3-diméthyl 5-hydroxy 6-aminoindole
le 2,3,5-triméthyl 6-aminoindole
le 2-méthyl 5-hydroxy 6-aminoindole
le 2,3-diméthyl 6-aminoindole
le 2,3,7-triméthyl 6-aminoindole
le 2,3,4-triméthyl 6-aminoindole.

**5.** Procédé selon la revendication 1 ou 2, caractérisé par le fait que le composé amino indoline de formule (III) est choisi parmi la 6-amino indoline ou la N-éthyl 6-amino indoline.

**6.** Procédé selon l'une quelconque des revendications 1 à 5, caractérisé par le fait que le composé de formule (I) est présent dans la composition (A) dans des proportions comprises entre 0,01 et 5% en poids par rapport au poids de la composition (A) ou au poids total de (A) + (B) et que le composé de formule (II) ou (III) est présent dans la composition (B) dans des proportions comprises entre 0,01 et 5% en poids par rapport au poids de la composition (B) ou du poids total de (A) + (B).

**7.** Procédé selon l'une quelconque des revendications 1 à 6, caractérisé par le fait que la composition (A) et/ou la composition (B) est une composition aqueuse ou anhydre sous forme liquide plus ou moins épaissi, une composition sous forme de crème, de gel aqueux ou anhydre, d'huile ou de poudre à diluer avec un liquide au moment de l'emploi.

**8.** Procédé selon la revendication 7, caractérisé par le fait que la composition (A) et/ou la composition (B) se présente sous forme d'une composition aqueuse ayant un pH compris entre 2 et 10 et contenant un ou plusieurs adjuvants cosmétiquement acceptables, choisis parmi les agents tensio-actifs anioniques, cationiques, non-ioniques ou leurs mélanges, des solvants organiques, des polymères anioniques, non-ioniques, cationiques, amphotères ou leurs mélanges, des agents épaississants, des agents de pénétration, des agents séquestrants, des agents antioxydants, des tampons, des colorants, des parfums.

**9.** Procédé selon la revendication 7, caractérisé par le fait que la composition (A) et/ou la composition (B) se présente sous forme d'une composition anhydre contenant un ou plusieurs solvants anhydres et un ou plusieurs tensio-actifs anhydres, dans des proportions d'au moins 15% de solvant et d'au moins 20% d'agent tensio-actif.

**10.** Procédé selon la revendication 9, caractérisé par le fait que le solvant anhydre est choisi parmi les monoalcools saturés en $C_2$-$C_{20}$, les polyols, les éthers de glycol, les esters de glycol, les esters d'acide gras d'alcools inférieurs.

**11.** Procédé selon la revendication 7, caractérisé par le fait que la composition (A) et/ou la composition (B) se présentent sous forme de poudre, à diluer avec un liquide au moment de l'emploi, constituée par des substances amylacées ou mucilagineuses ou par des poudres ou farines choisies parmi les silices, les argiles, les végétaux pulvérisés après extraction de leurs principes actifs par des solvants.

**12.** Procédé selon la revendication 11, caractérisé par le fait que l'on réalise un cataplasme à partir de la composition (A) et/ou la composition (B) sous forme de poudre, par addition d'un liquide cosmétiquement acceptable, dans des proportions suffisantes pour obtenir une viscosité de 0,3 à 5 Pa.s.

**13.** Procédé selon l'une quelconque des revendications 1 à 12, caractérisé par le fait que l'on mélange les composants (A) et (B) juste avant emploi, que l'on applique immédiatement la composition résultante sur les fibres kératiniques, qu'on laisse agir pendant 5 à 40 minutes; les fibres kératiniques étant ensuite rincées, lavées au shampooing, rincées à nouveau, puis séchées.

**14.** Procédé selon l'une quelconque des revendications 1 à 12, caractérisé par le fait qu'il comporte l'application sur les fibres kératiniques du composant (A) suivie ou précédée de l'application sur lesdites fibres du composant (B), que l'on laisse agir chaque composant pendant 5 à 40 minutes, que l'on procède éventuellement à un rinçage à l'eau entre chaque application; les fibres kératiniques étant ensuite rincées, lavées au shampooing, rincées à nouveau, puis séchées.

**15.** Agent de teinture des fibres kératiniques et en particulier des cheveux, caractérisé par le fait qu'il comporte les composants (A) et (B) tels que définis dans les revendications 1 à 12, sous forme séparée; les composants (A) et (B) étant destinés à être, soit mélangés tout juste avant emploi, soit appliqués de façon successive sur les fibres à traiter.

**16.** Dispositif à plusieurs compartiments ou "kit de teinture", caractérisé par le fait qu'il comporte au moins deux compartiments dont un renferme le composant (A) tel que défini dans l'une quelconque des revendications 1, 2, 6 à 12, et le second renferme le composant (B) tel que défini dans l'une quelconque des revendications 1, 3 à 12.

**17.** Dispositif selon la revendication 16, caractérisé par le fait que le composant (A) et/ou le composant (B) se présente sous forme de composition anhydre et qu'il comporte un troisième compartiment contenant un milieu aqueux cosmétiquement acceptable approprié pour la teinture destiné à être mélangé avant emploi dans l'un ou les deux premiers compartiments renfermant chaque composant (A) ou (B).

## Claims

1. Process for dyeing keratinous fibres, in particular human hair, characterised in that there is applied to the said fibres a component (A) consisting of a composition containing, in a medium suitable for dyeing, at least one compound of formula (I):

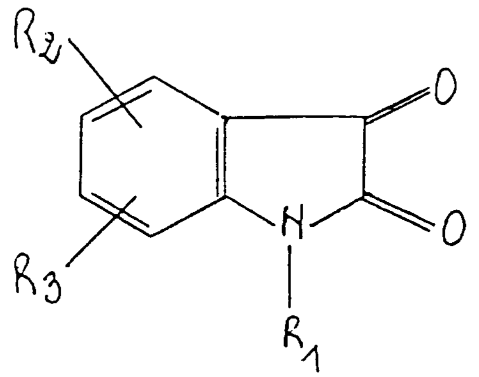

(I)

in which:

$R_1$ denotes a hydrogen atom, a $C_1$-$C_6$ alkyl radical, acetyl, benzoyl, phenyl or $C_1$-$C_4$ carboxyalkyl;

$R_2$ and $R_3$, independently of each other, denote a hydrogen atom, a $C_1$-$C_6$ alkyl, $C_1$-$C_4$ alkoxy, a hydroxyl, an amino, a halogen atom, a nitro, an alkyl($C_1$-$C_6$)phenyl, phenyl, alkyl($C_1$-$C_6$)amino, hydroxyalkyl($C_1$-$C_6$)amino, polyhydroxyalkyl($C_2$-$C_6$)amino group;

and a component (B) consisting of a composition containing, in a medium suitable for dyeing, at least one aminoindole compound of the following formula (II):

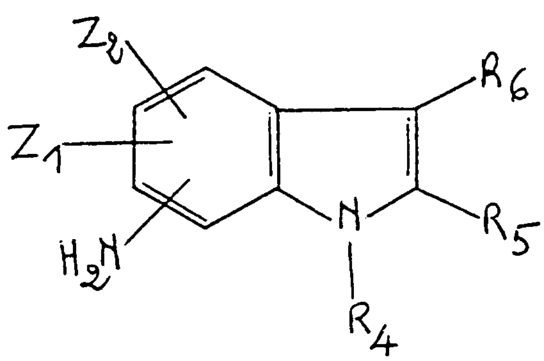

(II)

in which:

$R_4$ and $R_6$, independently of each other, represent a hydrogen atom or a $C_1$-$C_4$ alkyl group;

$R_5$ denotes a hydrogen atom or a $C_1$-$C_4$ alkyl group or a COOR' group, R' being a hydrogen atom or a $C_1$-$C_4$ alkyl group;

$Z_1$ represents a hydrogen or a halogen atom, a $C_1$-$C_4$-alkyl, $C_1$-$C_4$ alkoxy or hydroxyl radical;

$Z_2$ represents a hydrogen atom or a $C_1$-$C_4$ alkyl radical;

as well as its cosmetically acceptable salts, or alternatively an aminoindoline of formula (III):

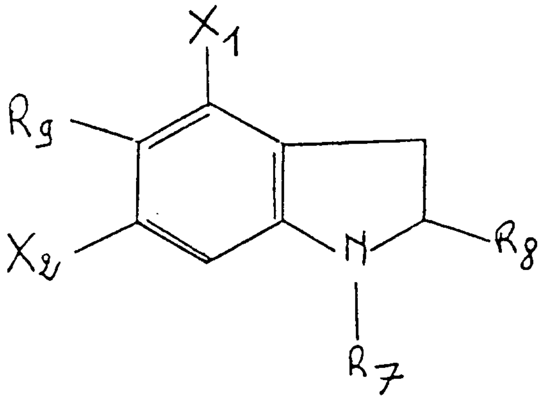

(III)

in which:

$R_7$ and $R_8$, independently of each other, represent a hydrogen atom or a $C_1$-$C_4$ alkyl radical;

$X_1$ and $X_2$ represent a hydrogen atom or an $NH_2$ radical, at least one, and only one, representing $NH_2$;

$R_9$ denotes a hydrogen atom, a $C_1$-$C_4$ alkyl or $C_1$-$C_4$-alkoxy radical; $R_9$ denoting hydrogen when $X_2$ denotes $NH_2$;

as well as its cosmetically acceptable salts.

**2.** Process according to Claim 1, characterised in that the compound of formula (I) is isatin.

**3.** Process according to Claim 1 or 2, characterised in that the compound of formula (II) is chosen from:
4-aminoindole
5-aminoindole
7-aminoindole
2,3-dimethyl-5-amino-6-hydroxyindole
2,3-dimethyl-5-amino-6-methoxyindole
2,3-dimethyl-5-chloro-6-aminoindole
2,3,4,5-tetramethyl-6-aminoindole
2,3-dimethyl-5-methoxy-6-aminoindole
2,3-dimethyl-5-ethyl-6-aminoindole
2-methyl-6-aminoindole.

**4.** Process according to Claim 1 or 2, characterised in that the compound of formula (II) is chosen from:
6-aminoindole
2,3-dimethyl-5-hydroxy-6-aminoindole
2,3,5-trimethyl-6-aminoindole
2-methyl-5-hydroxy-6-aminoindole
2,3-dimethyl-6-aminoindole
2,3,7-trimethyl-6-aminoindole
2,3,4-trimethyl-6-aminoindole.

**5.** Process according to Claim 1 or 2, characterised in that the aminoindoline compound of formula (III) is chosen from 6-aminoindoline or N-ethyl-6-aminoindoline.

**6.** Process according to any one of Claims 1 to 5, characterised in that the compound of formula (I) is present in the composition (A) in proportions of between 0.01 and 5% by weight relative to the weight of the composition (A) or to the total weight of (A) + (B) and in that the compound of formula (II) or (III) is present in the composition (B) in proportions of between 0.01 and 5% by weight relative to the weight of the composition (B) or of the total weight of (A) + (B).

**7.** Process according to any one of Claims 1 to 6, characterised in that the composition (A) and/or the composition (B) is an aqueous or anhydrous composition in more or less thickened liquid form, a

composition in the form of a cream, an aqueous or anhydrous gel, an oil or a powder to be diluted with a liquid at the time of use.

**8.** Process according to Claim 7, characterised in that the composition (A) and/or the composition (B) is present in the form of an aqueous composition having a pH of between 2 and 10 and containing one or more cosmetically acceptable adjuvants chosen from anionic, cationic or nonionic surface-active agents or their mixtures, organic solvents, anionic, nonionic, cationic or amphoteric polymers or their mixtures, thickening agents, penetrating agents, sequestering agents, antioxidants, buffers, dyes and perfumes.

**9.** Process according to Claim 7, characterised in that the composition (A) and/or the composition (B) is provided in the form of an anhydrous composition containing one or more anhydrous solvents and one or more anhydrous surface-active agents, in proportions of at least 15% of solvent and at least 20% of surface-active agent.

**10.** Process according to Claim 9, characterised in that the anhydrous solvent is chosen from $C_2$-$C_{20}$ saturated monoalcohols, polyols, glycol ethers, glycol esters, esters of fatty acids of (sic) lower alcohols.

**11.** Process according to Claim 7, characterised in that the composition (A) and/or the composition (B) is provided in the form of a powder, to be diluted with a liquid at the time of use, consisting of starchy or mucilaginous substances or of powders or flours chosen from silicas, clays, plants pulverised after extracting their active ingredients with solvents.

**12.** Process according to Claim 11, characterised in that a cataplasm is prepared from the composition (A) and/or the composition (B) in the form of a powder, by adding a cosmetically acceptable liquid in sufficient proportions in order to obtain a viscosity of 0.3 to 5 Pa.s.

**13.** Process according to any one of Claims 1 to 12, characterised in that the components (A) and (B) are mixed immediately before use, in that the resulting composition is immediately applied to keratinous fibres, in that it is allowed to act for 5 to 40 minutes; the keratinous fibres then being rinsed, washed with shampoo, rinsed again and then dried.

**14.** Process according to any one of Claims 1 to 12, characterised in that it comprises the application to the keratinous fibres of the component (A) followed or preceded by the application to the said fibres of the component (B) in that each component is allowed to act for 5 to 40 minutes, in that rinsing with water is optionally carried out between each application; the keratinous fibres then being rinsed, washed with shampoo, rinsed again and then dried.

**15.** Agent for dyeing keratinous fibres, and in particular hair, characterised in that it comprises the components (A) and (B) as defined in Claims 1 to 12 in separate form; the components (A) and (B) being intended to be either mixed immediately before use, or to be applied successively to the fibres to be treated.

**16.** Multi-compartment device or "dyeing kit", characterised in that it comprises at least two compartments, one of which contains the component (A) such as defined in any one of Claims 1, 2, 6 to 12, and the second contains the component (B) such as defined in any one of Claims 1, 3 to 12.

**17.** Device according to Claim 16, characterised in that the component (A) and/or the component (B) is provided in the form of an anhydrous composition and in that it comprises a third compartment containing a cosmetically acceptable aqueous medium suitable for dyeing, intended to be mixed before use in one or in the first two compartments containing each component (A) or (B).

**Patentansprüche**

**1.** Verfahren zur Färbung keratinischer Fasern, insbesondere menschlicher Haare,
dadurch **gekennzeichnet**, daß
man auf die genannten Fasern einen Bestandteil (A) aus einer Zusammensetzung, die in einem zur Färbung geeigneten Milieu mindestens eine Verbindung der folgenden Formel (I) enthält:

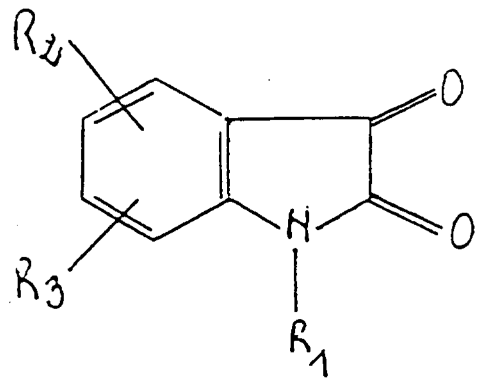

(I)

worin gilt:
$R_1$ bedeutet ein Wasserstoffatom, einen $C_{1-6}$-Alkyl-, Acetyl-, Benzyl-, Phenyl- oder einen $C_{1-4}$-Carboxyalkylrest;
$R_2$ und $R_3$ bedeuten, unabhängig voneinander, ein Wasserstoffatom, einen $C_{1-6}$-Alkyl-, $C_{1-4}$-Alkoxy-, Hydroxyl-, Aminorest, ein Halogenatom, eine Nitrogruppe, einen $C_{1-6}$-Alkylphenyl-, Phenyl-, $C_{1-6}$-Alkylamino-, Hydroxy-$C_{1-6}$-Alkylamino- oder einen Polyhydroxy-$C_{2-6}$-alkylaminorest;
und einen Bestandteil (B) aus einer Zusammensetzung aufbringt, die in einem zur Färbung geeigneten Milieu mindestens ein Aminoindol der folgenden Formel (II):

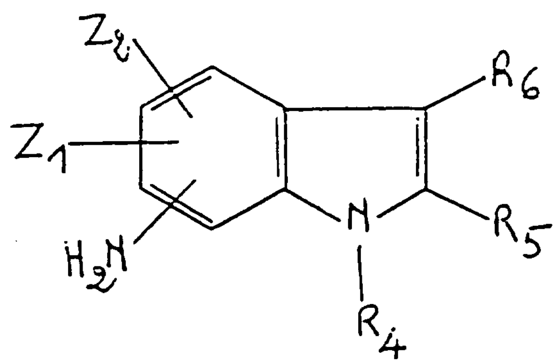

(II)

worin gilt:
$R_4$ und $R_6$ stellen, unabhängig voneinander ein Wasserstoffatom oder eine $C_{1-4}$-Alkylgruppe dar;
$R_5$ bedeutet ein Wasserstoffatom oder eine $C_{1-4}$-Alkyl- oder eine COOR'-Gruppe, worin R' ein Wasserstoffatom oder eine $C_{1-4}$-Alkylgruppe ist;
$Z_1$ stellt ein Wasserstoffatom, Halogenatom,
einen $C_{1-4}$-Alkyl-, $C_{1-4}$-Alkoxy- oder einen Hydroxylrest dar;
$Z_2$ stellt ein Wasserstoffatom oder einen $C_{1-4}$-Alkylrest dar, sowie dessen kosmetisch geeigneten Salze, oder auch ein Aminoindolin der Formel (III):

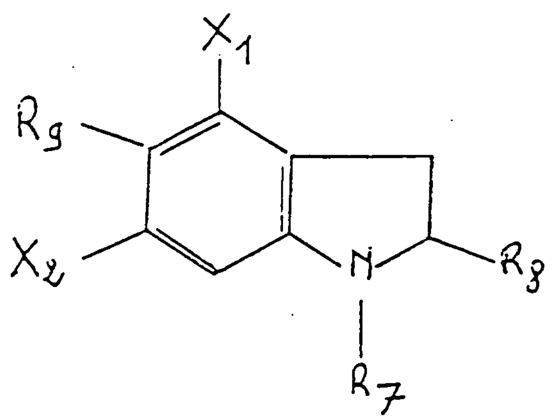

(III)

worin gilt:
$R_7$ und $R_8$ stellen, unabhängig voneinander, ein Wasserstoffatom oder einen $C_{1-4}$-Alkylrest dar;
$X_1$ und $X_2$ stellen ein Wasserstoffatom oder einen $NH_2$-Rest dar, wobei mindestens einer und nur einer $NH_2$ darstellen;
$R_9$ bedeutet ein Wasserstoffatom, einen $C_{1-4}$-Alkyl- oder $C_{1-4}$-Alkoxyrest, wobei $R_9$ Wasserstoff bedeutet, wenn $X_2$ $NH_2$ bedeutet,
sowie dessen kosmetisch geeigneten Salze,
enthält.

2. Verfahren gemäß Anspruch 1,
dadurch **gekennzeichnet**, daß
die Verbindung der Formel (I) Isatin ist.

3. Verfahren gemäß Anspruch 1 oder 2,
dadurch **gekennzeichnet**, daß
die Verbindung der Formel (II) ausgewählt ist aus: : 4-Aminoindol, 5-Aminoindol, 7-Aminoindol, 2,3-Dimethyl-5-amino-6-hydroxyindol, 2,3-Dimethyl-5-amino-6-methoxyindol, 2,3-Dimethyl-5-chlor-6-aminoindol, 2,3,4,5-Tetramethyl-6-aminoindol, 2,3-Dimethyl-5-methoxy-6-aminoindol, 2,3-Dimethyl-5-ethyl-6-aminoindol, 2-Methyl-6-aminoindol.

4. Verfahren gemäß Anspruch 1 oder 2,
dadurch **gekennzeichnet**, daß
die Verbindung der Formel (II) ausgewählt ist aus: 6-Aminoindol, 2,3-Dimethyl-5-hydroxy-6-aminoindol, 2,3,5-Trimethyl-6-aminoindol, 2-Methyl-5-hydroxy-6-aminoindol, 2,3-Dimethyl-6-aminoindol, 2,3,7-Trimethyl-6-aminoindol, 2,3,4-Trimethyl-6-aminoindol.

5. Verfahren gemäß Anspruch 1 oder 2,
dadurch **gekennzeichnet**, daß
die Aminoindolin-Verbindung der Formel (III) aus 6-Aminoindolin oder N-Ethyl-6-aminoindolin ausgewählt ist.

6. Verfahren gemäß jedem der Ansprüche 1 bis 5,
dadurch **gekennzeichnet**, daß
die Verbindung der Formel (I) in der Zusammensetzung (A) in Mengenanteilen von 0,01 bis 5 Gew.%, bezogen auf das Gewicht der Zusammensetzung (A) oder auf das Gesamtgewicht von (A) + (B), und daß die Verbindung der Formel (II) oder (III) in der Zusammensetzung (B) in Mengenanteilen von 0,01 bis 5 Gew.% vorhanden sind, bezogen auf das Gewicht der Zusammensetzung (B) oder auf das Gesamtgewicht von (A) + (B).

7. Verfahren gemäß jedem der Ansprüche 1 bis 6,
dadurch **gekennzeichnet**, daß
die Zusammensetzung (A) und/oder die Zusammensetzung (B) eine wässrige oder wasserfreie Zusammensetzung in flüssiger, mehr oder weniger verdickter Form, eine Zusammensetzung in Form einer Creme, eines wässrigen oder wasserfreien Gels, eines Öls oder eines zum Zeitpunkt der Anwendung mit einer Flüssigkeit zu verdünnenden Pulvers ist.

**8.** Verfahren gemäß Anspruch 7,
dadurch **gekennzeichnet**, daß
die Zusammensetzung (A) und/oder die Zusammensetzung (B) in Form einer wässrigen Zusammensetzung mit einem pH-Wert von 2 bis 10 vorliegt und einen oder mehrere kosmetisch geeignete Hilfsstoffe enthält, ausgewählt aus anionischen, kationischen oder nicht-ionischen oberflächenaktiven Mitteln oder deren Mischungen, aus organischen Lösungsmitteln, anionischen, nicht-ionischen, kationischen oder amphoteren Polymeren oder deren Mischungen, aus Verdickungsmitteln, Eindringmitteln, Sequestriermitteln, Antioxidantien, Puffermitteln, Farbstoffen und aus Parfüm-Produkten.

**9.** Verfahren gemäß Anspruch 7,
dadurch **gekennzeichnet**, daß
die Zusammensetzung (A) und/oder die Zusammensetzung (B) in Form einer wasserfreien Zusammensetzung vorliegen, die ein oder mehrere wasserfreie Lösungsmittel und ein oder mehrere wasserfreie oberflächenaktive Mittel in Mengenanteilen von wenigstens 15% Lösungsmittel und wenigestens 20% oberflächenaktives Mittel enthalten.

**10.** Verfahren gemäß Anspruch 9,
dadurch **gekennzeichnet**, daß
das wasserfreie Lösungsmittel aus gesättigten $C_{2-20}$-Monoalkoholen, Polyolen, Glycolethern, Glycolestern und aus Estern von Fettsäuren und Niedrigalkoholen ausgewählt ist.

**11.** Verfahren gemäß Anspruch 7,
dadurch **gekennzeichnet**, daß
die Zusammensetzung (A) und/oder die Zusammensetzung (B) in Form eines zum Zeitpunkt der Anwendung mit einer Flüssigkeit zu verdünnenden Pulvers vorliegen, das aus stärkehaltigen oder schleimartigen Substanzen oder aus Pulver- oder aus Mehlprodukten zusammengesetzt ist, die aus Kieselsäuren, Tonmaterialien und aus Pflanzenmaterialien ausgewählt sind, die nach einer mit Lösungsmitteln durchgeführten Extraktion von deren Wirkprinzipverbindungen pulverisiert sind.

**12.** Verfahren gemäß Anspruch 11,
dadurch **gekennzeichnet**, daß
man ein Kataplasma aus der Zusammensetzung (A) und/oder der Zusammensetzung (B) in Form eines Pulvers durch Zugabe einer kosmetisch geeigneten Flüssigkeit herstellt, wobei die Mengenverhältnisse so eingestellt werden, daß man eine Viskosität von 0,3 bis 5 Pa x s erhält.

**13.** Verfahren gemäß jedem der Ansprüche 1 bis 12,
dadurch **gekennzeichnet**, daß
man die Bestandteile (A) und (B) kurz vor der Anwendung vermischt, die entstandene Zusammensetzung sofort auf die keratinischen Fasern aufbringt, das Ganze 5 bis 40 Minuten lang einwirken läßt, worauf die keratinischen Fasern gespült, unter Schamponieren gewaschen, erneut gespült und dann getrocknet werden.

**14.** Verfahren gemäß jedem der Ansprüche 1 bis 12,
dadurch **gekennzeichnet**, daß
man auf die keratinischen Fasern den Bestandteil (A) vor oder nach der entsprechenden Aufbringung des Bestandteils (B) aufträgt, jeden Bestandteil 5 bis 40 Minuten lang einwirken läßt, man gegebenenfalls eine Spülung mit Wasser zwischen jeder Aufbringung durchführt, worauf die keratinischen Fasern gespült, unter Schamponieren gewaschen, erneut gespült und dann getrocknet werden.

**15.** Mittel zur Färbung keratinischer Fasern und insbesondere der Haare,
dadurch **gekennzeichnet**, daß
es die in jedem der Ansprüche 1 bis 12 definierten Bestandteile (A) und (B) in getrennter Form aufweist, wobei die Bestandteile (A) und (B) entweder kurz vor deren Anwendung vermischt oder nacheinander auf die zu behandelnden Fasern aufgebracht werden sollen.

**16.** Vorrichtung aus mehreren Teilstücken oder "Kit zur Färbung", dadurch **gekennzeichnet**, daß sie mindestens zwei Teilstücke umfassen, wovon das eine den in jedem der Ansprüche 1, 2 und 6 bis 12 definierten Bestandteil (A) und das zweite den in jedem der Ansprüche 1 und 3 bis 12 definierten Bestandteil (B) einschließen.

**17.** Vorrichtung gemäß Anspruch 16, dadurch **gekennzeichnet**, daß der Bestandteil (A) und/oder der Bestandteil (B) in Form einer wasserfreien Zusammensetzung vorliegen und ein drittes Teilstück umfassen, das ein kosmetisch geeignetes wässriges Milieu enthält, das sich zur Färbung eignet und dazu bestimmt ist, vor der Anwendung in dem einen oder den beiden ersteren Teilen vermischt zu werden, welche einen jeden Bestandteil (A) oder (B) einschließen.